# EUROPÄISCHE PATENTSCHRIFT

(11) **EP 3 024 812 B1**
(45) Veröffentlichungstag und Bekanntmachung des Hinweises auf die Patenterteilung: **02.01.2019**
(21) Anmeldenummer: 14741261.3
(22) Anmeldetag: 15.07.2014
(51) Int. Cl.: C07C 45/75

(54) **VERFAHREN ZUR REGULIERUNG DES WASSERGEHALTS IN EINEM KONTINUIERLICHEN VERFAHREN ZUR HERSTELLUNG VON METHACROLEIN**
METHOD OF REGULATING THE WATER CONTENT IN A CONTINUOUS METHOD FOR PRODUCING METHACROLEIN
PROCÉDÉ DE RÉGULATION DE LA TENEUR EN EAU DANS UN PROCÉDÉ CONTINU DE PRODUCTION DE MÉTHACROLÉINE

(30) Priorität: 24.07.2013 EP 13177889
(43) Veröffentlichungstag der Anmeldung: 01.06.2016
(73) Patentinhaber: Evonik Röhm GmbH, 64293 Darmstadt (DE)
(72) Erfinder: BURGHARDT, Rudolf, 64287 Darmstadt (DE); GLUTH, Frederik, 40474 Düsseldorf (DE); KÖLBL, Gerhard, 64579 Gernsheim (DE); KRILL, Steffen, 64367 Mühltal (DE); BALDUF, Torsten, 64319 Pfungstadt (DE)
(86) Internationale Anmeldenummer: PCT/EP2014/065065
(87) Internationale Veröffentlichungsnummer: WO 2015/010942

(56) Entgegenhaltungen:
- US-A- 4 408 079
- JIAN K ET AL: "Asymmetric PVDF hollow-fiber membranes for organic/water pervaporation separations", JOURNAL OF MEMBRANE SCIENCE, ELSEVIER, Bd. 135, Nr. 1, 12. November 1997 (1997-11-12), Seiten 41-53, XP004096369, ISSN: 0376-7388, DOI: 10.1016/S0376-7388(97)00117-8

## Beschreibung

Die vorliegende Erfindung betrifft die Regulierung des Wassergehalts in einem Verfahren zur Herstellung von Methacrolein. Methacrolein wird in der chemischen Synthese insbesondere als Zwischenprodukt zur Herstellung von Methacrylsäure, Methylmethacrylat oder auch von Wirk-, Geruchs- oder Geschmacksstoffen verwendet. Insbesondere betrifft die vorliegende Erfindung die Regulierung des Wassergehalts in einem Verfahren zur Herstellung von Methacrolein aus Formaldehyd und Propionaldehyd mittels einer Mannich-Kondensation.

Es besteht ein großes Interesse an möglichst einfachen, wirtschaftlichen und umweltschonenden Herstellungsverfahren für Methacrolein.

### Stand der Technik

Es sind diverse aminkatalytische Prozesse bekannt insbesondere Verfahren zur Herstellung von Methacrolein, bei denen aminhaltiges Reaktionswasser anfällt. Ein großtechnisch bedeutendes Verfahren zur Herstellung von Methacrolein geht von Propanal und Formaldehyd aus und erfolgt über eine Mannich-Reaktion. Ein solches Verfahren zur Herstellung von Methacrolein, ist unter anderem in den Druckschriften US 7,141,702, US 4,408,079, JP 3069420, JP 4173757, EP 0 317 909 und US 2,848,499 beschrieben.

Von besonderem Interesse ist es dabei naturgemäß, diese Reaktion kontinuierlich durchzuführen. Dabei fällt als Nebenprodukt der Mannich-Reaktion oder einer Aldolähnlichen-Addition aminhaltiges Wasser an. Dieses Wasser stellt einerseits das Reaktionsmedium und Lösungsmittel für Edukte und Katalysatorlösung dar, und wird insbesondere zur Moderierung der Reaktionswärme und der Reaktionsführung benötigt. Auf der anderen Seite wird in einem solchen Prozess das Wasser, z.B. den Katalysator enthaltend, zurückgeführt. Ein Nachteil dieses Vorgehens ist jedoch, dass sich mit der Zeit das Wasser in dem Reaktionskreislauf anreichert und entfernt werden muss. Nach der weitgehenden Entfernung vom Reaktionsprodukt, dem Methacrolein, wird das Wasser zusammen mit aktiven und inaktiven Katalysatorbestandteilen einer Entsorgung zugeführt.

Zu berücksichtigen ist weiter, dass auch mit den Ausgangsmaterialien sowie optional mit den Katalysatorkomponenten Wasser in die Reaktion gelangen kann und dort den Wasserhaushalt mit beeinflusst.

So gelangen mit dem eingesetzten Formalin erhebliche Mengen Wasser ins System; je nach gewählter Stärke bzw. Konzentration des Formalins, üblicherweise werden handels- und produktionsübliche Formalinkonzentrationen zwischen 36 - 60 wt% Formaldehyd in Wasser eingesetzt, ergibt sich so per se eine Grundlast an Wasser, die nach Abtrennung vom Wertprodukt Methacrolein zusammen mit dem während der Reaktion gebildeten Wasser behandelt werden muss. Weiterhin muss bezüglich dem Wasserhaushalt in Reaktion und Aufarbeitung berücksichtigt werden, dass auch die Nebenreaktionen Wasser freisetzen. Dies gilt sowohl für die Nebenreaktionen des Propanals als auch für die Nebenprodukte der katalytisch aktiven Amine. Die katalytisch aktiven Amine bilden unter den Reaktionsbedingungen nach Eschweiler-Clarke bzw. in einer Leukart Wallach ähnlichen Reaktion höher-alkylierte Derivate, die teilweise nicht mehr katalytisch aktiv sind. So bildet sich beispielhaft aus Dimethylamin durch Reaktion mit einem Formaldehyd Equivalent Trimethylamin, während Wasser freigesetzt wird.

Auf der anderen Seite wird in einem solchen Prozess das Wasser, z.B. den Katalysator enthaltend, zurückgeführt. Ein Nachteil dieses Vorgehens ist jedoch, dass sich mit der Zeit das Wasser in dem Reaktionskreislauf anreichert und entfernt werden muss. Wenn man dagegen auf die Zurückführung verzichtet, muss am Anfang der Reaktion neuer Katalysator zugegeben werden. Dies macht das Verfahren weniger wirtschaftlich und die Abwässer ökologisch bedenklich. Weiterhin sind in der wässrigen Phase auch gewisse Mengen nicht abgetrenntes Methacrolein enthalten, das auf diese Weise dem System entzogen wird und damit die Gesamtausbeute des Verfahrens mindert. Eine Rückführung der wässrigen Phase wird beispielsweise in JP 4173757A (JP 19900300135) beschrieben.

In DE 32 13 681 werden diesbezüglich mehrere Alternativen vorgeschlagen. Bei geringen Katalysatormengen in der wässrigen Phase, bzw. dem Destillationssumpf, der neben der wässrigen Phase und dem Katalysator zusätzlich hochsiedende Nebenprodukte und restliche Monomere und Methacrolein enthält, wird vorgeschlagen diesen zu entsorgen. Bei höheren Konzentrationen wird vorgeschlagen, den Sumpf einer weiteren, sehr aufwendigen Destillation zu unterziehen, um die Wassermenge zu reduzieren. Der Rest würde dann zurück in den Reaktionsraum geführt. Dieses Vorgehen ist jedoch nicht nur energetisch ungünstig, sondern es werden bei einer solchen zweiten Destillation zugleich ausbeutesenkend Methacrolein verworfen, welches aufgrund des geringeren Siedepunktes mit abdestilliert würde.

In einer dritten Alternative der DE 32 13 681 wird der Sumpf geteilt und ein Teil derart in den Reaktionsraum zurückgeführt, dass der Wassergehalt derart zunimmt und beeinflusst werden kann. Der andere Teil des Sumpfes - und damit der wässrigen Phase - wird verworfen. Dieses Vorgehen hat jedoch den Nachteil, dass der verworfene Anteil neben Produkt auch Katalysatormengen enthält, die dem System zum Ausgleich frisch wieder zugeführt werden müssen. Damit sinkt zum einen die Ausbeute und auf der anderen Seite steigt der Katalysatorverbrauch.

### Aufgabe

In Anbetracht des Standes der Technik war es daher Aufgabe der vorliegenden Erfindung, den Wassergehalt in einer kontinuierlich betriebenen Reaktion, bei der Reaktionswasser gebildet wird, zu regulieren. Insbesondere war es Aufgabe den Wassergehalt in einer kontinuierlichen Mannich-Reaktion zu regulieren.

Insbesondere war es Aufgabe der vorliegenden Erfindung, den Wassergehalt in einer kontinuierlich betriebenen Mannich-Reaktion zur Herstellung von Methacrolein zu regulieren, wobei einem in den Reaktionsraum zurückgeführten Destillationssumpf nur Wasser und geringe Mengen anderer Bestandteile entzogen werden.

Weiterhin bestand eine zusätzliche Aufgabe, das Wasser derart zu entziehen, dass es danach einer biologischen Aufarbeitung zur Verfügung steht und eine andere, zur Verbrennung in einem Thermal Oxidizer ausgeschleuste, Hochsieder enthaltende Phase mit einem geringeren Wassergehalt erhalten wird.

Ferner sollte das Verfahren mit relativ einfachen und kostengünstigen Modifikationen bestehender Anlagen realisiert werden können. Die Modifikationen sollten demgemäß mit geringen Investitionskosten verbunden sein. Hierbei sollten die Anlagen auch nach der Modifikation einfach zu warten sein und geringe Unterhaltskosten verursachen. Insbesondere ist es Aufgabe, ein Verfahren zur Verfügung zu stellen, das gegenüber dem Stand der Technik einen verminderten Energiebedarf aufweist und einen geringeren Katalysatoraustrag ermöglicht.

Weitere nicht explizit genannte Aufgaben ergeben sich aus dem Gesamtzusammenhang der nachfolgenden Beschreibung und der Ansprüche.

### Lösung

Gelöst werden diese Aufgaben mittels eines neuartigen Verfahrens, das zur kontinuierlichen Durchführung einer Mannich-Reaktion geeignet ist. Dieses Verfahren zeichnet sich dadurch aus, dass eine wässrige, mindestens einen Aminkatalysator enthaltende Phase, mittels mindestens einer Membrantrennstufe in einen überwiegend aus Wasser bestehenden Strom und einen im Wassergehalt reduzierten Strom getrennt wird.

Das Reaktionsprodukt der Reaktion kann vorher z.B. in einer Kolonne zumindest teilweise abgetrennt werden. In diesem Fall wird der wasserhaltige Austrag, der beispielsweise unterhalb des Zulaufes dieser Kolonne entnommen wird, anschließend mittels mindestens einer Membrantrennstufe getrennt.

Besonders bevorzugt wird der wasserhaltige Austrag der Kolonne nach der Entnahme geteilt. Dabei werden ein erster Teil dieser Entnahme auf die Membrantrennstufe und der andere Teil zurück in den Reaktionsraum geleitet. Auf diese Weise ist es möglich, den Wassergehalt im Reaktionsraum konstant zu halten. Insbesondere bevorzugt wird der in den Reaktionsraum zurückgeleitete Strom konstant gehalten. Entsprechend kann der auf die Membranstufe geleitete Teil bzw. Strom bezüglich des Volumens je nach Sumpfmenge, insbesondere Wassermenge in der Kolonne variieren.

Alternativ zu dieser bevorzugten Ausführungsform sind jedoch auch andere Ausführungsformen möglich. So kann beispielsweise das Reaktionsprodukt auch in einem Phasentrenner von der wässrigen, mindestens einen Aminkatalysator enthaltenden Phase getrennt werden. In einem solchen Fall wird diese wässrige Phase ganz oder teilweise zur Membrantrennstufe geleitet und das Reaktionsprodukt beispielsweise in eine Destillationskolonne überführt. Der Sumpf dieser Kolonne wiederum kann dann zurück in den Phasentrenner, auf die Membrantrennstufe, zurück in den Reaktionsraum oder direkt zu einer, z.B. thermischen Entsorgung geleitet werden.

Bevorzugt sind die Membranen der Membrantrennstufe dergestalt ausgewählt, dass es sich bei dem überwiegend aus Wasser bestehenden Strom um das Permeat und dem im Wassergehalt reduzierten Strom um das Retentat der Membrantrennstufe handelt. Es ist jedoch auch möglich, Membranen einzusetzen, bei denen es sich bei dem im Wassergehalt reduzierten Strom um das Permeat und bei dem überwiegend aus Wasser bestehenden Strom um das Retentat handelt.

Diese erste bevorzugte Ausführungsform, bei der es sich bei dem überwiegend aus Wasser bestehenden Strom um das Permeat und dem im Wassergehalt reduzierten Strom um das Retentat der Membrantrennstufe handelt, wird zur leichteren Verständnis der Erfindung im Weiteren beispielhaft beschrieben. Es sei jedoch explizit darauf hingewiesen, dass auch die weniger bevorzugte, umgekehrte Ausführungsform, bei der es sich bei dem im Wassergehalt reduzierten Strom um das Permeat und bei dem überwiegend aus Wasser bestehenden Strom um das Retentat handelt, von der weiteren Beschreibung, wenn nicht explizit anders ausgeführt, jeweils mit umfasst ist.

Bevorzugt handelt es sich bei der vorgeschalteten Mannich-Reaktion um eine Umsetzung von einem Aldehyd mit 2 bis 6 Kohlenstoffatomen mit Formaldehyd zu einem ungesättigten Aldehyd in einem Reaktionsraum. Besonders bevorzugt handelt es sich um eine Mannich-Reaktion, bei der Propanal mit Formaldehyd zu Methacrolein umgesetzt wird.

Insbesondere zeichnet sich diese Reaktion dadurch aus, dass die Umsetzung in Gegenwart von 0,1 bis 20 Mol% organische Base, bevorzugt einem sekundären Amin, und 0,1 bis 20 Mol% Säure, bevorzugt eine organische Säure, jeweils bezogen auf das Aldehyd mit 2 bis 6 Kohlenstoffatomen, bei einer Temperatur von 100 bis 300 °C und bei einem Druck von 5 bis 100 bar durchgeführt wird. Druck und Temperatur werden in der Regel so eingestellt, dass die Umsetzung stets unterhalb des Siedepunktes des Reaktionsgemisches erfolgt, die Reaktion also in flüssiger Phase verläuft.

Die zur Herstellung von Methacrolein geeignete, auf einer Mannich-Reaktion basierenden Verfahren sind dem Fachmann bekannt und Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2. Insbesondere bezieht sich das erfindungsgemäß vor der Zuleitung des aminhaltigen Reaktionswassers besonders bevorzugt durchgeführte Verfahren auf eine kontinuierlich durchgeführte Mannich-Reaktion wie sie in der europäischen Patentanmeldung mit dem Anmeldeaktenzeichen 13002076.1 offenbart ist. Erfindungsgemäß wird zur Verdeutlichung einer solchen bevorzugten Vorstufe auf den Offenbarungsgehalt dieser Anmeldung bezüglich der Methacrolein-Synthese Bezug genommen.

Bei den Säuren handelt es sich in der Regel um anorganische Säuren oder organische Mono-, Di- bzw. Polycarbonsäuren, bevorzugt Monocarbonsäuren, insbesondere aliphatische Monocarbonsäuren. Besonders bevorzugt wird zur Umsetzung von Propanal und Formaldehyd mindestens eine organische Säure, besonders bevorzugt Essigsäure eingesetzt. Der Anteil an Säure beträgt zwischen 0,1 und 20, vorteilhaft von 0,5 bis 10, bevorzugt 1 bis 5 Mol-%, bezogen auf Propanal.

Bei den organischen Basen handelt es sich vorzugsweise um Amine, besonders bevorzugt um sekundäre Amine. Als Amine kommen beispielsweise in Betracht: Dimethylamin, Diethylamin, Methylethylamin, Methylpropylamin, Dipropylamin, Dibutylamin, Di-iso-propylamin, Di-iso-butylamin, Methyl-iso-propylamin, Methyl-iso-butylamin, Methyl- sek.-butylamin, Methyl-(2-methylpentyl)-amin, Methyl-(2-ethylhexyl)-amin, Pyrrolidin, Piperidin, Morpholin, N-Methylpiperazin, N-Hydroxyethylpiperazin, Piperazin, Hexamethylenimin, Diethanolamin, Methylethanolamin, Methylcyclohexylamin, Methylcyclopentylamin, Dicyclohexylamin oder entsprechende Gemische. Der Anteil an organischer Base beträgt zwischen 0,1 und 20, vorteilhaft von 0,5 bis 10, bevorzugt 1 bis 5 Mol-%, bezogen auf Propanal.

Das Verhältnis der Äquivalente Amin zu Säure wird vorzugsweise so gewählt, dass im Reaktionsgemisch vor der Reaktion ein pH-Wert von 2,5 bis 9 resultiert.

In dem aminhaltigen Reaktionswasser können neben den genannten Komponenten und Nebenprodukten zusätzlich verbliebene Edukte oder deren Folgeprodukte wie Paraformaldehyd, oder organische Lösungsmittel wie z.B. Propanol, Dioxan, Tetrahydrofuran, Methoxyethanol enthalten sein. Insbesondere können auch Nebenprodukte des Katalysatorsystems, wie zum Beispiel tertiäre Amine oder deren Salze enthalten sein.

In einem solchen Verfahren erfolgt die Abtrennung des Produktes wie z.B. des Methacroleins - wie beschrieben - bevorzugt über eine Destillationskolonne. In der unteren Hälfte einer solchen Kolonne befindet sich der Zulauf. Unten in dieser Kolonne sammelt sich ein Sumpf, der überwiegend aus dem Reaktionswasser der Reaktion besteht. Dieses enthält zusätzlich die Katalysatorkomponenten, wie zum Beispiel die organische Säure und das sekundäre Amin bzw. das aus diesen gebildete Salz, sowie Nebenprodukte der Reaktion. Diese wässrige Katalysatorlösung kann unterhalb des Zulaufs, insbesondere am Sumpf der Kolonne abgezogen werden. Das Reaktionswasser setzt sich dabei aus dem Wasser, welches als Katalysatorlösung zugegeben wurde, dem bei der Reaktion gebildeten Wasser und optional dem Wasser der Formaldehydlösung zusammen.

Bei einer Aldol-Kondensation oder einer Mannich-Reaktion, wie zum Beispiel der Herstellung von Methacrolein aus Propanal und Formaldehyd, wird bevorzugt das Reaktionsgemisch einer Kolonne zugeleitet und dort mit Wasserdampf gestrippt. Das Produkt verlässt zusammen mit Wasser die Kolonne am Kopf. Das Gemisch wird kondensiert und über ein Phasentrenngefäß in eine obere und eine untere Phase getrennt. Die obere Phase enthält das Produkt, zum Beispiel Methacrolein. Die untere Phase besteht hauptsächlich aus Wasser. Vorzugsweise kann dieses Wasser zur Entfernung des noch darin gelösten Produktes zumindest teilweise, bevorzugt ganz, wieder in die Kolonne zurückgeführt werden.

Ein bei der Herstellung von Methacrolein aus Propanal und Formaldehyd erzeugtes Reaktionswasser setzt sich aus dem Wasser, welches als Katalysatorlösung zugegeben wurde, dem bei der Reaktion gebildeten und optional dem aus der Formaldehydlösung zusammen. Weitere, jedoch in geringerem Umfang zu berücksichtigende Wasserquellen sind Bestandteile der technischen Edukte wie Propional und Wasser, das in diversen Nebenreaktionen der Katalysatorkomponten mit Edukten, Nebenprodukten und Reaktionsprodukten gebildet wird, sowie Reaktionswasser aus allen diesen Komponenten, die unter den Reaktionsbedingungen entstehen. In diesem aminhaltigen Reaktionswasser können neben den genannten Komponenten und Nebenprodukten zusätzlich verbliebene Edukte oder deren Folgeprodukte enthalten sein. Insbesondere sind dabei die Katalysatorkomponenten, wie ein sekundäres Amin und eine organische Säure, sowie das daraus gebildete Salz zu nennen. Nebenprodukte dieser Katalysatoren sind bezüglich einer Entsorgung von besonderem Interesse. Hier sind als Beispiel vor allem höher alkylierte Amine, wie insbesondere Trimethylamin bei Verwendung von Dimethylamin als originärem Katalysatoramin aufzuführen. Auch kleine Mengen Edukte oder Produkt können in dem aminhaltigen Reaktionswasser enthalten sein. Beispiele hierfür sind Methacrolein, Formaldehyd, Paraformaldehyd und Propanal. Als Nebenprodukte der Reaktion, die gleichsam in dem aminhaltigen Reaktionswasser enthalten sind, wären beispielsweise Dimere, Oligomere oder Polymere des Methacrolein zu nennen. Weiterhin können je nach Prozessführung auch weitere Hilfsstoffe, wie organische Lösungsmittel, wie z.B. Methanol, Ameisensäure, Propanol, Dioxan, Tetrahydrofuran oder Methoxyethanol enthalten sein - sowie weitere in der Reaktionsmatrize enthaltende oder entstehende Stoffe.

In einer besonderen, bevorzugten Ausführungsform der Erfindung wird der überwiegend aus Wasser bestehende Strom, bevorzugt das Permeat der ersten Membrantrennstufe mittels einer zweiten Membrantrennstufe in einen zweiten, überwiegend aus Wasser bestehenden Strom, bevorzugt in ein zweites, wasserhaltiges Permeat und einen zweiten im Wassergehalt reduzierten Strom, bevorzugt ein zweites Retentat getrennt. Dieses zweite Permeat zeichnet sich dadurch aus, dass es einen sehr hohen Wassergehalt und einen sehr geringen Anteil an Katalysatorkomponenten, Produkt, Edukten und Nebenprodukten aufweist. Somit ist es zumeist möglich, diese Permeat einer biologischen Entsorgung zuzuführen. Eine solche Entsorgung wäre mit dem Abwasser einer analogen, nicht erfindungsgemäßen Anlage nicht möglich. Hier müsste ein zusätzlicher Destillationsschritt zur Gewinnung eines weniger belasteten Wassers erfolgen oder aber das Abwasser müsste einer thermischen Entsorgung, z.B. in einem Thermal Oxidizer zugeführt werden.

Selbst in Bezug auf das erste Permeat der ersten Membrantrennstufe aus dem erfindungsgemäßen Verfahren kann es bereits möglich sein, dass dieses einer biologischen Aufarbeitung zugeführt werden kann. Dies hängt von der Fahrweise der Anlage und von der daraus resultierenden Belastung des Permeats mit den genannten Stoffen ab. Unabhängig davon ist ein solches Permeat weniger mit diesen Stoffen belastet als ein aus der Anlage ausgeschleuster Kolonnensumpf. Damit wäre auch ein stärker belastetes Permeat einfacher mittels einer Destillation aufzureinigen.

Weiterhin hat das erfindungsgemäße Verfahren, unabhängig von der Ausführungsform mit einer oder zwei Membrantrennstufen, den großen Vorteil gegenüber dem Stand der Technik, dass im Sumpf der Kolonne gelöste Katalysatorkomponenten auf diese Weise zu einem Großteil in der Anlage verbleiben. Dies ist sehr vorteilhaft gegenüber einer reinen Entnahme des Sumpfes zur Reduzierung des Wassergehalts. Das gleiche gilt auch für im Sumpf gelöstes Produkt. Da dieses an den Membrantrennstufen nicht mit ausgeschleust wird, wird die Gesamtausbeute des Prozesses mit dem erfinderischen Verfahren sogar erhöht.

In diesem erfindungsgemäßen Verfahren können beispielsweise Membranen der Nanofiltration oder Umkehrosmose, die zur Abtrennung von Wasser aus einer Mischung geeignet sind, verwendet werden. Dazu bekannt sind Membranen aus dem Bereich der Wasseraufarbeitung bzw. Wasserentsalzung, wie sie sonst zum Beispiel zur Gewinnung von Trinkwasser oder Kesselspeisewasser in Kraftwerken Verwendung finden. Bevorzugt handelt es sich um Membranen, die eine trennaktive Schicht aus Polyamid, Celluloseacetat oder Polyethersulfon, besonders bevorzugt aus einem Polyamid aufweisen. Ein gut geeignetes Beispiel dafür ist die Dow Filmtec SW30HR Membran.

In der Regel liegen die Membranen als Spiralwickelelemente vor. Der genaue Aufbau solcher Membranen kann beispielsweise in Th. Melin, R. Rautenbach "Membranverfahren - Grundlagen der Modul- und Anlagenauslegung", 3. Auflage, Springer Verlag, Berlin, S. 173-175 nachgelesen werden.

Erfindungsgemäß bevorzugt weist die Membrantrennstufe eine lokale Temperatur an der Membran zwischen 10 und 70 °C, bevorzugt zwischen 30 und 40 °C auf. Gleichfalls bevorzugt liegt der Transmembrandruck zwischen 20 und 100 bar, bevorzugt zwischen 50 und 90 bar und besonders bevorzugt zwischen 70 und 90 bar. Die genaue Anlagentechnik zur Inkludierung der der Membrantrennstufen in eine solche Produktionsanlage ist dem Fachmann bekannt und kann beispielsweise in Th. Melin, R. Rautenbach "Membranverfahren - Grundlagen der Modul- und Anlagenauslegung", 3. Auflage, Springer Verlag, Berlin, S. 205-226 und 245-308 nachgelesen werden.

Das erste Retentat, das an der ersten bzw. einzigen Membrantrennstufe gewonnen wird, wird bevorzugt ganz oder teilweise in den Reaktionsraum zurückgeführt. Teilweise bedeutet in diesem Fall, dass ein Teil des Retentats zur Entfernung von Nebenprodukten, die sich sonst in der Anlage anreichern würden, ausgeschleust wird. Dies kann kontinuierlich erfolgen. Optional erfolgt dieses Ausschleusen batchweise. So kann eine Ausschleusung z.B. in regelmäßigen Abständen erfolgen. Besonders bevorzugt wird mittels inline-Sonden der Gehalt solcher Nebenprodukte bestimmt. In diesem Fall kann das Ausschleusen bei Überschreiten eines vorher festgelegten Grenzwertes erfolgen. Je nach Art des Nebenproduktes kann es sich bei diesen Sonden um eine pH-Wert-Bestimmung, eine IR- oder RI-Sonde, eine Viskositätsbestimmung oder Leitfähigkeitsmessung handeln. Auch ist es möglich entsprechende Daten von den Temperatursonden in der Destillationskolonne zu erhalten. Es ist jedoch auch einfach möglich, die ausgeschleuste Wassermenge des Permeats hinter den Membrantrennstufen zu messen. Auch kann in diesem ausgeschleusten Wasser, die Konzentration an mit ausgeschleustem Wasser bestimmt werden. Gleichfalls kann auch die Menge ausgeschleuster Katalysatorkomponenten, bzw. deren Folgeprodukte im Retentat bestimmt werden. Damit können die entstehenden Nebenprodukte sehr einfach aus den Reaktionsmischungen abgetrennt werden, so dass das Verfahren insgesamt mit einer hohen Ausbeute durchgeführt werden kann, ohne dass aufwendige Reinigungsschritte notwendig sind.

Zusätzlich kann der Wassergehalt und/oder Katalysatorgehalt in der Produktionsanlage bestimmt werden. Dies kann beispielsweise in der Leitung zur Zurückführung des Retentats in den Reaktionsraum erfolgen. Insbesondere nach einer Entnahme von Retentat aus erläuterten Gründen ist ein Ausgleich der Katalysatorkonzentration, z.B. durch Zugabe einer organischen Säure und eines Amins nötig. Nach Bedarf kann dann auf Basis dieser Messwerte Katalysator in den Reaktionsraum zugeführt werden.

Weiterhin ist es möglich, dass über das ausgeschleuste Permeat der Anlage mehr Wasser entzogen wird, als bei der Reaktion gebildet wird bzw. durch die Edukt- und Katalysatorzugabe der Anlage zugeführt wird. Dafür wird bevorzugt in der Leitung zur Zurückführung des Retentats oder direkt in dem Reaktionsraum der Wassergehalt online mittels einer dazu geeigneten Sonde bestimmt. Besonders bevorzugt wird einfach die Menge des durch die Membrantrennstufen abgeführten Wassers bestimmt. Durch Zugabe weiteren Wassers kann dann die Anlage mit einer konstanten Wasserkonzentration betrieben werden.

Bevorzugt ist der Wassergehalt im Eingang des Reaktionsraumes nicht größer als 75 % der Gesamtmasse.

Wie bereits erläutert, kann ein Retentat einer Verbrennung, z.B. in einem Thermal Oxidizer, zugeführt werden. Insbesondere kann das zweite Retentat entweder einer Verbrennung und/oder der ersten Membrantrennstufe und/oder dem Reaktionsraum zugeführt wird. Auf diese Weise erhält man eine sehr effiziente Wasserentnahme und im Falle einer Zurückführung nur sehr geringe Mengen an Abfallflüssigkeit, die verbrannt werden muss. Weiterhin hat diese dem Thermal Oxidizer zuzuführende Phase gegenüber dem Stand der Technik einen deutlich geringeren Wassergehalt und kann daher deutlich energiesparender verbrannt werden.

Das erste Retentat wird dagegen bevorzugt entweder zurück in den Reaktionsraum und/oder, besonders bevorzugt - wie erläutert - nur bei Bedarf in eine Verbrennung geleitet.

Weiterhin ist es in einer weiteren Ausführungsform der Erfindung auch möglich, die Anlage so zu gestalten, dass der wässrige Austrag aus dem unteren Kolonnenteil der Destillationsskolonne entweder zur ersten Membrantrennstufe und/oder anteilig zurück in den Reaktionsraum geleitet wird. Dies kann einerseits kontinuierlich über eine einfache Trennung des Stroms in Teilströme erfolgen. Dabei kann ein Teilstrom gerade die Menge an Wasser mit sich führen, die nötig ist, über die Membrantrennstufen die Menge Wasser auszuschleusen, die bei der Reaktion gebildet und mit den Ausgangsstoffen eingegeben wurde. Dieser Teilstrom wird dann ausgeschleust und auf die Membrantrennstufe geleitet. Der andere Teilstrom wird in den Reaktor zurückgeführt.

Andererseits kann der Fluss auch je nach Bedarf gesteuert werden. So ist es zum Beispiel möglich, die Anlage beim Anfahren ausschließlich unter Rückführung in den Reaktionsraum zu betreiben und erst nach der Ansammlung einer größeren Wassermenge in der Anlage den Sumpf auf die Membrantrennstufe zu leiten. Auch kann eine solche Umschaltung, z.B. in Form eines Ventils oder eines Dreiwegehahns, auch mittels Sonden erfasster Daten bezüglich des Wassergehalts und/oder der Nebenproduktkonzentration automatisch gesteuert werden.

Ein großer Vorteil der vorliegenden Erfindung ist, dass das Verfahren mit relativ einfachen und kostengünstigen Modifikationen bestehender Anlagen durchgeführt werden kann. Die Modifikationen sind mit geringen Investitionskosten verbunden. Hierbei sind die Anlagen einfach zu warten und verursachen geringe Unterhaltskosten. Zur Wartung kann zum Beispiel der Strom an der jeweiligen Membrantrennstufe vorbeigeführt werden. So ist es möglich, die jeweilige Membrantrennstufe einer Reinigung, zum Beispiel mit Membranreinigungsmitteln, zu unterziehen, während die eigentliche Produktionsanlage weiter betrieben wird. Weiterhin ist es beispielsweise auch möglich, mehrere Membranen in einer Membrantrennstufe parallel anzuordnen. Mit einer solchen Ausführung der Erfindung ist es dann sogar möglich, die Membrantrennstufe weiter zu nutzen, während einzelne Membranen offline geschaltet sind und gereinigt werden.

### Bezugszeichenliste zu den Zeichnungen

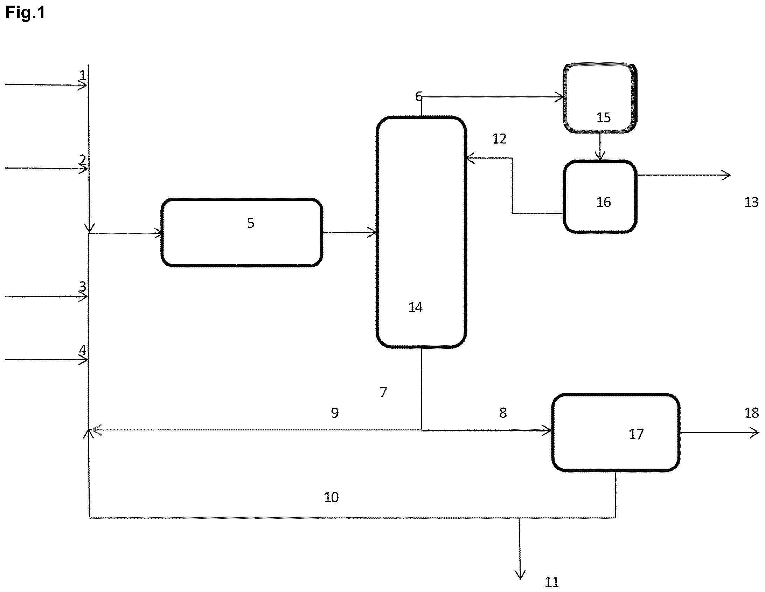

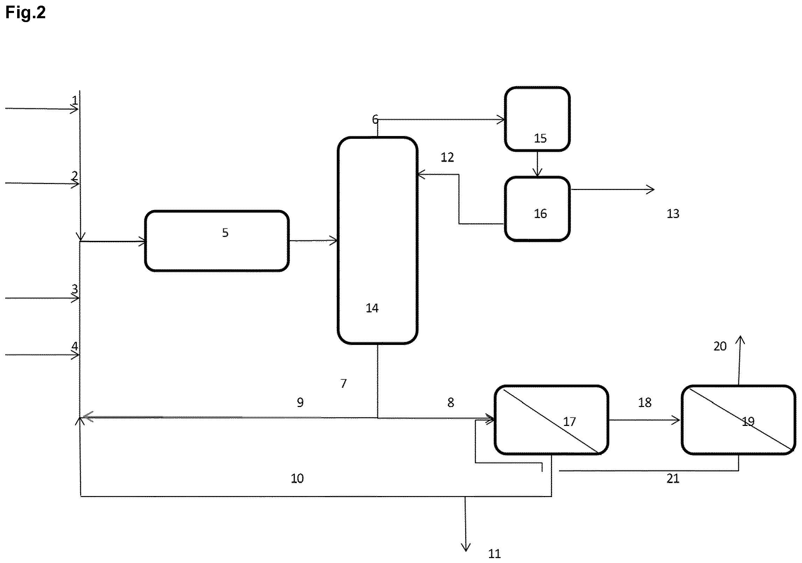

Fig.1: Ausführungsform mit einer Membranstufe
   1 Formalin Dosierung
   2 Propionaldehyd Dosierung
   3 Dimethylamin Dosierung
   4 Essigsäure Dosierung
   5 Reaktor bzw. Reaktionsraum
   6 Kopfprodukt Kolonne
   7 Sumpfprodukt
   8 Zulauf Membrantrennstufe
   9 Rücklauf Kolonnensumpf zum Reaktor
   10 Rücklauf Retentat Membranstufe zum Reaktor
   11 Aussschleusung Retentat
   12 Rücklauf wässrige Phase zur Kolonne
   13 Methacrolein Produkt
   14 Destillationskolonne
   15 Kondensator
   16 Phasentrenner
   17 Membrantrennstufe
   18 Permeat
Fig.2: Ausführungsform mit zwei Membrantrennstufen
   1 Formalin Dosierung
   2 Propionaldehyd Dosierung
   3 Dimethylamin Dosierung
   4 Essigsäure Dosierung
   5 Reaktor bzw. Reaktionsraum
   6 Kopfprodukt Kolonne
   7 Sumpfprodukt
   8 Zulauf Membrantrennstufe
   9 Rücklauf Kolonnensumpf zum Reaktor
   10 Rücklauf Retentat Membranstufe zum Reaktor
   11 Aussschleusung Retentat
   12 Rücklauf wässrige Phase zur Kolonne
   13 Methacrolein Produkt
   14 Destillationskolonne
   15 Kondensatoren
   16 Phasentrenner
   17 1. Membrantrennstufe
   18 Permeat 1. Membranstufe zu 2. Membrantrennstufe
   19 2. Membrantrennstufe
   20 Permeat 2. Membrantrennstufe
   21 Retentat 2. Membrantrennstufe zur 1. Membrantrennstufe

## Patentansprüche

1. Verfahren zur kontinuierlichen Durchführung einer Mannich-Reaktion, **dadurch gekennzeichnet, dass** eine wässrige, mindestens einen Aminkatalysator enthaltende Phase, mittels mindestens einer Membrantrennstufe in einen überwiegend aus Wasser bestehenden Strom und einen im Wassergehalt reduzierten Strom getrennt wird.

2. Verfahren gemäß Anspruch 1, **dadurch gekennzeichnet, dass** das Reaktionsprodukt in einer Kolonne zumindest teilweise abgetrennt wird, und dass ein wasserhaltiger Austrag, der unterhalb des Zulaufes dieser Kolonne entnommen wird, anschließend zumindest teilweise mittels mindestens einer Membrantrennstufe getrennt wird.

3. Verfahren gemäß Anspruch 2, **dadurch gekennzeichnet, dass** der wasserhaltige Austrag geteilt wird und ein Teil auf die Membrantrennstufe und der andere Teil zurück in den Reaktionsraum geleitet wird.

4. Verfahren gemäß mindestens einem der Ansprüche 1 bis 3, **dadurch gekennzeichnet, dass** es sich bei dem überwiegend aus Wasser bestehenden Strom um das Permeat und dem im Wassergehalt reduzierten Strom um das Retentat der Membrantrennstufe handelt.

5. Verfahren gemäß mindestens einem der Ansprüche 1 bis 4, **dadurch gekennzeichnet, dass** in einem Reaktionsraum ein Aldehyd mit 2 bis 6 Kohlenstoffatomen, bevorzugt Propanal mit Formaldehyd zu einem ungesättigten Aldehyd, bevorzugt zu Methacrolein umgesetzt wird.

6. Verfahren gemäß Anspruch 5, **dadurch gekennzeichnet, dass** die Umsetzung in Gegenwart von 0,1 bis 20 Mol% organische Base, bevorzugt einem sekundären Amin, und 0,1 bis 20 Mol% Säure, jeweils bezogen auf das Aldehyd mit 2 bis 6 Kohlenstoffatomen, bei einer Temperatur von 100 bis 300 °C und bei einem Druck von 5 bis 100 bar durchgeführt wird.

7. Verfahren gemäß mindestens einem der Ansprüche 1 bis 6, **dadurch gekennzeichnet, dass** der überwiegend aus Wasser bestehende Strom mittels einer zweiten Membrantrennstufe in einen zweiten, überwiegend aus Wasser bestehenden Strom und einen zweiten im Wassergehalt reduzierten Strom getrennt wird.

8. Verfahren gemäß mindestens einem der Ansprüche 1 bis 7, **dadurch gekennzeichnet, dass** die Membranen eine trennaktive Schicht aus Polyamid, Celluloseacetat oder Polyethersulfon aufweisen.

9. Verfahren gemäß mindestens einem der Ansprüche 1 bis 8, **dadurch gekennzeichnet, dass** die lokale Temperatur an der Membran zwischen 10 und 70 °C beträgt und der Transmembrandruck zwischen 20 und 100 bar liegt.

10. Verfahren gemäß mindestens einem der Ansprüche 1 bis 9, **dadurch gekennzeichnet, dass** der erste im Wassergehalt reduzierte Strom ganz oder teilweise in den Reaktionsraum zurückgeführt wird.

11. Verfahren gemäß Anspruch 10, **dadurch gekennzeichnet, dass** der Wassergehalt und/oder Katalysatorgehalt in der Produktionsanlage bestimmt wird und bei Bedarf zusätzliches Wasser und/oder Katalysator in den Reaktionsraum zugeführt werden.

12. Verfahren gemäß Anspruch 11, **dadurch gekennzeichnet, dass** der Wassergehalt und/oder Katalysatorgehalt mittels einer Sonde online oder durch Messung des durch die Membrantrennstufen abgeführten Wassers bestimmt wird.

13. Verfahren gemäß mindestens einem der Ansprüche 1 bis 12, **dadurch gekennzeichnet, dass** der Wassergehalt im Eingang des Reaktionsraumes nicht größer als 75 % der Gesamtmasse ist.

14. Verfahren gemäß mindestens einem der Ansprüche 1 bis 12, **dadurch gekennzeichnet, dass** mindestens ein durch die Membrantrennstufen abgetrennter, überwiegend aus Wasser bestehender Strom einer biologischen Aufarbeitung zugeführt wird.

15. Verfahren gemäß Anspruch 7, **dadurch gekennzeichnet, dass** der durch die zweite Membrantrennstufe abgetrennte, überwiegend aus Wasser bestehende Strom einer biologischen Aufarbeitung zugeführt wird.

16. Verfahren gemäß mindestens einem der Ansprüche 1 bis 15, **dadurch gekennzeichnet, dass** ein im Wassergehalt reduzierter Strom einer Verbrennung zugeführt wird.

17. Verfahren gemäß Anspruch 1 bis 16, **dadurch gekennzeichnet, dass** der zweite im Wassergehalt reduzierte Strom entweder einer Verbrennung und/oder der ersten Membrantrennstufe und/oder dem Reaktionsraum zugeführt wird.

18. Verfahren gemäß Anspruch 1 bis 17, **dadurch gekennzeichnet, dass** der erste im Wassergehalt reduzierte Strom entweder zurück in den Reaktionsraum und/oder in eine Verbrennung geleitet wird.

19. Verfahren gemäß mindestens einem der Ansprüche 1 bis 18, **dadurch gekennzeichnet, dass** der wässrige Austrag aus dem unteren Kolonnenteil der Destillationskolonne entweder zur ersten Membrantrennstufe und/oder zurück in den Reaktionsraum geleitet wird.

## Claims

1. Process for continuous operation of a Mannich reaction, **characterized in that** an aqueous phase which contains at least one amine catalyst is separated by at least one membrane separation stage into a stream consisting predominantly of water and a stream of reduced water content.

2. Process according to Claim 1, **characterized in that** the reaction product is at least partially separated off in a column and **in that** a water-containing effluent is withdrawn underneath the feedpoint for this column and is subsequently separated at least partially by at least one membrane separation stage.

3. Process according to Claim 2, **characterized in that** the water-containing effluent is divided and one portion is directed onto the membrane separation stage and the other portion is directed back into the reaction space.

4. Process according to at least one of Claims 1 to 3, **characterized in that** the stream which consists predominantly of water is the permeate of the membrane separation stage and the stream of reduced water content is the retentate of the membrane separation stage.

5. Process according to at least one of Claims 1 to 4, **characterized in that** an aldehyde of 2 to 6 carbon atoms, preferably propanal, is reacted with formaldehyde in a reaction space to form an unsaturated aldehyde, preferably methacrolein.

6. Process according to Claim 5, **characterized in that** the reaction is carried out in the presence of 0.1 to 20 mol% of an organic base, preferably a secondary amine, and 0.1 to 20 mol% of an acid, each based on the aldehyde of 2 to 6 carbon atoms, at a temperature of 100 to 300°C and a pressure of 5 to 100 bar.

7. Process according to at least one of Claims 1 to 6, **characterized in that** the stream which consists predominantly of water is separated by a second membrane separation stage into a second stream consisting predominantly of water and a second stream of reduced water content.

8. Process according to at least one of Claims 1 to 7, **characterized in that** the membranes comprise a separation-active layer of polyamide, cellulose acetate or polyether sulphone.

9. Process according to at least one of Claims 1 to 8, **characterized in that** the local temperature at the membrane is between 10 and 70°C and the transmembrane pressure is between 20 and 100 bar.

10. Process according to at least one of Claims 1 to 9, **characterized in that** the first stream of reduced water content is wholly or partly returned into the reaction space.

11. Process according to Claim 10, **characterized in that** the water content and/or catalyst content in the production plant is determined and the additional water and/or catalyst is as necessary fed into the reaction space.

12. Process according to Claim 11, **characterized in that** the water content and/or catalyst content is determined using an on-line probe or by measuring the water removed by the membrane separation stages.

13. Process according to at least one of Claims 1 to 12, **characterized in that** the water content in the inlet to the reaction space is not greater than 75% of the overall mass.

14. Process according to at least one of Claims 1 to 12, **characterized in that** at least one stream removed by the membrane separation stages which consists predominantly of water is sent for a biological work-up.

15. Process according to Claim 7, **characterized in that** the stream which was removed by the second membrane separation stage and consists predominantly of water is sent for a biological work-up.

16. Process according to at least one of Claims 1 to 15, **characterized in that** a stream of reduced water content is sent for incineration.

17. Process according to Claim 1 to 16, **characterized in that** the second stream of reduced water content is sent either for incineration and/or to the first membrane separation stage and/or to the reaction space.

18. Process according to Claim 1 to 17, **characterized in that** the first stream of reduced water content is directed either back into the reaction space and/or into an incineration.

19. Process according to at least one of Claims 1 to 18, **characterized in that** the aqueous effluent from the bottom part of the distillation column is directed either to the first membrane separation stage and/or back into the reaction space.

## Revendications

1. Procédé de réalisation continue d'une réaction de Mannich, **caractérisé en ce qu'**une phase aqueuse, contenant au moins un catalyseur aminé, est séparée au moyen d'au moins une étape de séparation sur membrane en un courant essentiellement constitué par de l'eau et un courant à teneur réduite en eau.

2. Procédé selon la revendication 1, **caractérisé en ce que** le produit de réaction est au moins partiellement séparé dans une colonne, et **en ce qu'**une sortie contenant de l'eau, qui est soutirée en dessous de l'alimentation de cette colonne, est ensuite au moins partiellement séparée au moyen d'au moins une étape de séparation sur membrane.

3. Procédé selon la revendication 2, **caractérisé en ce que** la sortie contenant de l'eau est divisée, et une partie est acheminée dans l'étape de séparation sur membrane et l'autre partie est recyclée dans la chambre de réaction.

4. Procédé selon au moins l'une quelconque des revendications 1 à 3, **caractérisé en ce que** le courant essentiellement constitué par de l'eau est le perméat et le courant à teneur réduite en eau est le rétentat de l'étape de séparation sur membrane.

5. Procédé selon au moins l'une quelconque des revendications 1 à 4, **caractérisé en ce qu'**un aldéhyde contenant 2 à 6 atomes de carbone, de préférence le propanal, est mis en réaction avec du formaldéhyde dans une chambre de réaction pour former un aldéhyde insaturé, de préférence de la méthacroléine.

6. Procédé selon la revendication 5, **caractérisé en ce que** la réaction est réalisée en présence de 0, 1 à 20 % en moles d'une base organique, de préférence d'une amine secondaire, et de 0, 1 à 20 % en moles d'un acide, à chaque fois par rapport à l'aldéhyde contenant 2 à 6 atomes de carbone, à une température de 100 à 300 °C et à une pression de 5 à 100 bar.

7. Procédé selon au moins l'une quelconque des revendications 1 à 6, **caractérisé en ce que** le courant essentiellement constitué par de l'eau est séparé au moyen d'une deuxième étape de séparation sur membrane en un deuxième courant essentiellement constitué par de l'eau et un deuxième courant à teneur réduite en eau.

8. Procédé selon au moins l'une quelconque des revendications 1 à 7, **caractérisé en ce que** les membranes comprennent une couche à activité de séparation en polyamide, acétate de cellulose ou polyéthersulfone.

9. Procédé selon au moins l'une quelconque des revendications 1 à 8, **caractérisé en ce que** la température locale sur la membrane est comprise entre 10 et 70 °C et la pression transmembranaire est comprise entre 20 et 100 bar.

10. Procédé selon au moins l'une quelconque des revendications 1 à 9, **caractérisé en ce que** le premier courant à teneur réduite en eau est recyclé en totalité ou en partie dans la chambre de réaction.

11. Procédé selon la revendication 10, **caractérisé en ce que** la teneur en eau et/ou la teneur en catalyseur dans l'unité de production sont déterminées, et de l'eau et/ou du catalyseur supplémentaires sont introduits au besoin dans la chambre de réaction.

12. Procédé selon la revendication 11, **caractérisé en ce que** la teneur en eau et/ou la teneur en catalyseur sont déterminées au moyen d'une sonde en ligne ou par mesure de l'eau déchargée par les étapes de séparation sur membrane.

13. Procédé selon au moins l'une quelconque des revendications 1 à 12, **caractérisé en ce que** la teneur en eau à l'entrée de la chambre de réaction n'est pas supérieure à 75 % de la masse totale.

14. Procédé selon au moins l'une quelconque des revendications 1 à 12, **caractérisé en ce qu'**au moins un courant essentiellement constitué par de l'eau séparé par les étapes de séparation sur membrane est introduit dans un traitement biologique.

15. Procédé selon la revendication 7, **caractérisé en ce que** le courant essentiellement constitué par de l'eau séparé par la deuxième étape de séparation sur membrane est introduit dans un traitement biologique.

16. Procédé selon au moins l'une quelconque des revendications 1 à 15, **caractérisé en ce qu'**un courant à teneur réduite en eau est introduit dans une combustion.

17. Procédé selon les revendications 1 à 16, **caractérisé en ce que** le deuxième courant à teneur réduite en eau est introduit dans une combustion et/ou dans la première étape de séparation sur membrane et/ou dans la chambre de réaction.

18. Procédé selon les revendications 1 à 17, **caractérisé en ce que** le premier courant à teneur réduite en eau est recyclé dans la chambre de réaction et/ou acheminé dans une combustion.

19. Procédé selon au moins l'une quelconque des revendications 1 à 18, **caractérisé en ce que** la sortie aqueuse de la partie inférieure de la colonne de distillation est acheminée dans la première étape de séparation sur membrane et/ou recyclée dans la chambre de réaction.
